(19) Europäisches Patentamt / European Patent Office / Office européen des brevets

(11) **EP 3 515 307 B1**

(12) **FASCICULE DE BREVET EUROPEEN**

(45) Date de publication et mention
de la délivrance du brevet:
**13.09.2023 Bulletin 2023/37**

(21) Numéro de dépôt: **17783918.0**

(22) Date de dépôt: **19.09.2017**

(51) Classification Internationale des Brevets (IPC):
*A61B 5/145* (2006.01)    *A61M 5/172* (2006.01)
*A61B 5/00* (2006.01)    *G16H 20/17* (2018.01)
*G16H 40/63* (2018.01)    *G16H 50/20* (2018.01)
*A61M 5/142* (2006.01)

(52) Classification Coopérative des Brevets (CPC):
**A61B 5/4839; A61B 5/14532; A61M 5/142;
A61M 5/1723; G16H 20/17; G16H 40/63;
G16H 50/20;** A61B 2560/0223; A61M 2005/14208;
A61M 2005/1726; A61M 2205/52; A61M 2230/201

(86) Numéro de dépôt international:
**PCT/FR2017/052511**

(87) Numéro de publication internationale:
**WO 2018/055283 (29.03.2018 Gazette 2018/13)**

(54) **SYSTÈME AUTOMATISÉ DE RÉGULATION DE LA GLYCÉMIE D'UN PATIENT**

AUTOMATISIERTES SYSTEM ZUR KONTROLLE DES BLUTZUCKERSPIEGELS EINES
PATIENTEN

AUTOMATED SYSTEM FOR CONTROLLING THE BLOOD GLUCOSE LEVEL OF A PATIENT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **21.09.2016 FR 1658881**

(43) Date de publication de la demande:
**31.07.2019 Bulletin 2019/31**

(73) Titulaire: **Commissariat à l'énergie atomique
et aux énergies alternatives
75015 Paris (FR)**

(72) Inventeur: **JALLON, Pierre
38700 Corenc (FR)**

(74) Mandataire: **Cabinet Beaumont
4, Place Robert Schuman
B.P. 1529
38025 Grenoble Cedex 1 (FR)**

(56) Documents cités:
**GB-A- 2 436 873    US-A1- 2014 066 884**

• **ROMAN HOVORKA ET AL: "Nonlinear model
predictive control of glucose concentration in
subjects with type 1 diabetes; Controlling
glucose", PHYSIOLOGICAL MEASUREMENT,
INSTITUTE OF PHYSICS PUBLISHING, BRISTOL,
GB, vol. 25, no. 4, 1 août 2004 (2004-08-01), pages
905-920, XP020074167, ISSN: 0967-3334, DOI:
10.1088/0967-3334/25/4/010**
• **J B Rawlings ET AL: "Model Predictive Control:
Theory and Design", , 19 août 2012 (2012-08-19),
XP055375565, Extrait de l'Internet:
URL:http://jbrwww.che.wisc.edu/home/jbraw/
mpc/electronic-book.pdf [extrait le 2017-05-24]**

EP 3 515 307 B1

**Description**

<u>Domaine</u>

La présente demande concerne le domaine des systèmes automatisés de régulation de glycémie, aussi appelés pancréas artificiels.

<u>Exposé de l'art antérieur</u>

Un pancréas artificiel est un système permettant de réguler automatiquement les apports en insuline d'un patient diabétique à partir de son historique de glycémie, de son historique de prise de repas, et de son historique d'injection d'insuline.

On s'intéresse ici plus particulièrement aux systèmes de régulation de type MPC (de l'anglais "Model-based Prédictive Control"), aussi appelés systèmes à commande prédictive, dans lesquels la régulation de la dose d'insuline administrée tient compte d'une prédiction de l'évolution future de la glycémie du patient, réalisée à partir d'un modèle physiologique décrivant l'assimilation de l'insuline par le corps du patient et son impact sur la glycémie du patient.

Les documents GB 2 436 873 A et "Nonlinear model prédictive control of glucose concentration in subjects with type 1 diabètes" (ROMAN HOVORKA ET AL, PHYSIOLOGICAL MEASUREMENT, vol. 25, no. 4, août 2004, pages 905-920, XP020074167, ISSN: 0967-3334, DOI:10.1088/0967-3334/25/4/010) décrivent des systèmes ou méthodes visant à la régulation de la glycémie d'un patient.

Il serait souhaitable de pouvoir améliorer les performances des pancréas artificiels à commande prédictive, et, plus particulièrement, de pouvoir améliorer la qualité de la prédiction de la glycémie future du patient, de façon à pouvoir contrôler avec une plus grande pertinence les apports en insuline et limiter les risques de placer le patient en situation d'hyperglycémie ou d'hypoglycémie.

Il serait par ailleurs souhaitable de pouvoir limiter les risques pour le patient liés à une éventuelle défaillance du modèle physiologique utilisé pour prédire la glycémie future du patient.

<u>Résumé</u>

L'invention est définie par les revendications jointes. Ainsi, l'invention prévoit un système automatisé de régulation de la glycémie d'un patient, comportant :

un capteur de glycémie ;
un dispositif d'injection d'insuline ; et
une unité de traitement et de contrôle adaptée à prédire l'évolution future de la glycémie du patient à partir d'un modèle physiologique et à commander le dispositif d'injection d'insuline en tenant compte de cette prédiction, dans lequel :

le modèle physiologique comporte un système d'équations différentielles décrivant l'évolution d'une pluralité de variables d'état en fonction du temps ; et
l'unité de traitement et de contrôle est adaptée à mettre en oeuvre une étape de calibration automatique du modèle physiologique comprenant une étape d'estimation de valeurs initiales des variables d'état par minimisation d'une grandeur représentative de l'erreur, pendant une période d'observation passée, entre la glycémie estimée à partir du modèle physiologique et la glycémie mesurée par le capteur.

Selon un mode de réalisation, la grandeur est représentative de l'aire entre une première courbe g représentative de l'évolution temporelle de la glycémie estimée à partir du modèle sur la période d'observation, et une deuxième courbe g représentative de l'évolution temporelle de la glycémie mesurée par le capteur sur la période d'observation.

Selon un mode de réalisation, la grandeur est définie comme suit :

$$m = \frac{1}{\Delta T} \sum_{t=t_0}^{t_0+\Delta T} |g(t) - \hat{g}(t)|^2$$

où t est une variable temps discrétisée, $t_0$ est l'instant de début de la phase d'observation, et $t_0+\Delta T$ est l'instant de fin de la phase d'observation.

Selon l'invention, le procédé de calibration comprend en outre une étape d'estimation de paramètres du système d'équations différentielles par minimisation de ladite grandeur.

Selon invention, le procédé de calibration comprend une pluralité d'itérations successives des étapes a) et b) suivantes :

a) estimer les paramètres du système d'équations différentielles par minimisation de ladite grandeur en fixant les valeurs initiales des variables d'état ; et

b) estimer les valeurs initiales des variables d'état par minimisation de ladite grandeur en fixant les paramètres du système d'équation différentielles.

Selon un mode de réalisation, à la première itération de l'étape a), les valeurs initiales des variables d'état sont déterminées analytiquement en faisant l'hypothèse que toutes les dérivées du système d'équations différentielles sont nulles.

Selon un mode de réalisation, pour simuler l'évolution de la glycémie du patient à partir du modèle physiologique, l'unité de traitement et de contrôle tient compte de l'historique d'insuline injectée au patient par le dispositif d'injection et de l'historique de glucose ingéré par le patient.

Selon un mode de réalisation, le modèle physiologique est le modèle de Hovorka.

Un autre aspect de l'invention prévoit un procédé de régulation automatisée de la glycémie d'un patient, comportant :

une étape de calcul, au moyen d'une unité de traitement et de contrôle, d'une prédiction de l'évolution future de la glycémie du patient à partir d'un modèle physiologique comportant un système d'équations différentielles décrivant l'évolution d'une pluralité de variables d'état en fonction du temps ;

une étape de commande d'un dispositif d'injection d'insuline en tenant compte de cette prédiction ; et

une étape de calibration automatique du modèle physiologique comprenant une étape d'estimation de valeurs initiales des variables d'état par minimisation d'une grandeur représentative de l'erreur, pendant une période d'observation passée, entre la glycémie estimée à partir du modèle physiologique et la glycémie mesurée sur le patient par un capteur de glycémie.

Selon l'invention, le procédé comporte en outre une étape d'estimation de paramètres du système d'équations différentielles par minimisation de ladite grandeur.

Selon l'invention, l'étape de calibration comprend une pluralité d'itérations successives des étapes a) et b) suivantes :

a) estimer les paramètres du système d'équations différentielles par minimisation de ladite grandeur en fixant les valeurs initiales des variables d'état ; et

b) estimer les valeurs initiales des variables d'état par minimisation de ladite grandeur en fixant les paramètres du système d'équations différentielles.

Un autre mode de réalisation non couvert par l'invention revendiquée prévoit un système automatisé de régulation de la glycémie d'un patient, comportant :

un capteur de glycémie ;

un dispositif d'injection d'insuline ; et

une unité de traitement et de contrôle adaptée à prédire l'évolution future de la glycémie du patient à partir d'un modèle physiologique et à commander le dispositif d'injection d'insuline en tenant compte de cette prédiction, dans lequel l'unité de traitement et de contrôle est adaptée à :

a) mettre en oeuvre une étape de calibration automatique du modèle physiologique en tenant compte d'un historique de glycémie mesurée par le capteur au cours d'une période d'observation passée ;

b) à l'issue de l'étape de calibration, déterminer si le modèle est satisfaisant ou non à partir d'au moins un indicateur numérique représentatif de l'erreur entre la glycémie estimée à partir du modèle et la glycémie réelle mesurée par le capteur ; et

c) si la qualité du modèle n'est pas satisfaisante, commander le dispositif d'injection d'insuline sans tenir compte de la prédiction réalisée à partir du modèle.

Selon un mode de réalisation, l'indicateur numérique comprend un indicateur m représentatif de l'aire entre une première courbe $\hat{g}$ représentative de l'évolution temporelle de la glycémie estimée à partir du modèle sur la période d'observation, et une deuxième courbe g représentative de l'évolution temporelle de la glycémie mesurée par le capteur sur la période d'observation.

Selon un mode de réalisation, l'indicateur m est défini comme suit :

$$m = \frac{1}{\Delta T} \sum_{t=t_0}^{t_0 + \Delta T} |g(t) - \hat{g}(t)|^2$$

où t est une variable temps discrétisée, $t_0$ est l'instant de début de la phase d'observation, et $t_0 + \Delta T$ est l'instant de fin de la phase d'observation.

Selon un mode de réalisation, l'indicateur numérique comprend un indicateur $m_1$ représentatif de la différence entre la glycémie estimée à partir du modèle et la glycémie mesurée par le capteur à un instant donné.

Selon un mode de réalisation, l'indicateur numérique comprend un indicateur $m_2$ représentatif de la différence entre la dérivée de la glycémie estimée à partir du modèle et la dérivée de la glycémie mesurée par le capteur à un instant donné.

Selon un mode de réalisation, à l'étape c), la commande du dispositif d'injection d'insuline est une commande prédictive basée sur un modèle physiologique simplifié.

Selon un mode de réalisation, à l'étape c), le dispositif d'injection d'insuline est commandé pour délivrer des doses d'insuline préprogrammées correspondant à un débit basal de référence prescrit au patient.

Selon un mode de réalisation, le modèle physiologique comporte un système d'équations différentielles décrivant l'évolution d'une pluralité de variables d'état en fonction du temps, et l'étape a) de calibration automatique du modèle comprend une étape d'estimation de paramètres du système d'équations différentielles par minimisation d'une grandeur représentative de l'erreur, pendant une période d'observation passée, entre la glycémie estimée à partir du modèle physiologique et la glycémie mesurée par le capteur.

Selon un mode de réalisation, l'étape a) de calibration automatique du modèle comprend en outre une étape de détermination de valeurs initiales des variables d'état.

Un autre mode de réalisation non couvert par l'invention revendiquée prévoit un procédé de régulation automatisée de la glycémie d'un patient, comportant :

une étape de calcul, au moyen d'une unité de traitement et de contrôle, d'une prédiction de l'évolution future de la glycémie du patient à partir d'un modèle physiologique ; et
une étape de commande d'un dispositif d'injection d'insuline en tenant compte de cette prédiction, ce procédé comportant en outre :

a) une étape de calibration automatique du modèle physiologique en tenant compte d'un historique de glycémie mesurée par un capteur de glycémie au cours d'une période d'observation passée ;
b) à l'issue de l'étape de calibration, une étape de détermination de la qualité du modèle physiologique à partir d'au moins un indicateur numérique représentatif de l'erreur entre la glycémie estimée à partir du modèle et la glycémie réelle mesurée par le capteur ; et
c) si la qualité du modèle est jugée insatisfaisante, une étape de commande du dispositif d'injection d'insuline sans tenir compte de la prédiction réalisée à partir du modèle.

Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :

la figure 1 représente de façon schématique, sous forme de blocs, un exemple d'un mode de réalisation d'un système automatisé de régulation de la glycémie d'un patient ;
la figure 2 est une représentation simplifiée d'un modèle physiologique utilisé dans le système de la figure 1 pour prédire l'évolution future de la glycémie du patient ;
la figure 3 est un diagramme représentant plus en détail un exemple de réalisation du modèle physiologique de la figure 2 ;
la figure 4 est un diagramme illustrant un exemple d'un procédé automatisé de régulation de glycémie mis en oeuvre par le système de la figure 1 ;
la figure 5 est un diagramme illustrant un exemple d'un mode de réalisation d'un procédé automatisé de calibration mis en oeuvre par le système de la figure 1 ; et

la figure 6 est un diagramme illustrant un exemple d'un mode de réalisation d'un procédé automatisé de régulation de glycémie mis en oeuvre par le système de la figure 1.

Description détaillée

De mêmes éléments ont été désignés par de mêmes références aux différentes figures. Par souci de clarté, seuls les éléments qui sont utiles à la compréhension des modes de réalisation décrits ont été représentés et sont détaillés. En particulier, le dispositif de mesure de glycémie et le dispositif d'injection d'insuline du système de régulation décrit n'ont pas été détaillés, les modes de réalisation décrits étant compatibles avec tous ou la plupart des dispositifs de mesure de glycémie et d'injection d'insuline connus. De plus, la réalisation matérielle de l'unité de traitement et de contrôle du système de régulation décrit n'a pas été détaillée, la réalisation d'une telle unité de traitement et de contrôle étant à la portée de l'homme du métier à partir des indications fonctionnelles décrites.

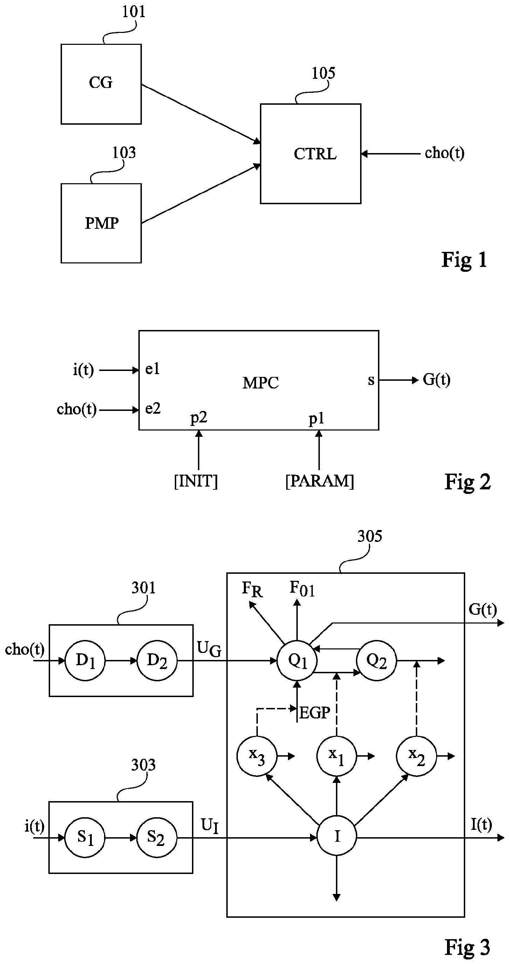

La figure 1 représente de façon schématique, sous forme de blocs, un exemple d'un mode de réalisation d'un système automatisé de régulation de la glycémie d'un patient.

Le système de la figure 1 comprend un capteur 101 (CG) adapté à mesurer la glycémie du patient. En fonctionnement normal, le capteur 101 peut être positionné à demeure sur ou dans le corps du patient, par exemple à hauteur de son abdomen. Le capteur 101 est par exemple un capteur de type CGM (de l'anglais "Continuous Glucose Monitoring" - surveillance continue de glycémie), c'est-à-dire un capteur adapté à mesurer en continu (par exemple au moins une fois par minute) la glycémie du patient. Le capteur 101 est par exemple un capteur de glycémie en sous cutané.

Le système de la figure 1 comprend en outre un dispositif d'injection d'insuline 103 (PMP), par exemple un dispositif d'injection en sous cutané. Le dispositif 103 est par exemple un dispositif d'injection automatique de type pompe à insuline, comportant un réservoir d'insuline relié à une aiguille d'injection implantée sous la peau du patient, la pompe pouvant être commandée électriquement pour injecter automatiquement des doses d'insuline déterminées à des instants déterminés. En fonctionnement normal, le dispositif d'injection 103 peut être positionné à demeure dans ou sur le corps du patient, par exemple au niveau de son abdomen.

Le système de la figure 1 comprend en outre une unité de traitement et de contrôle 105 (CTRL) reliée d'une part au capteur de glycémie 101, par exemple par liaison filaire ou par liaison radio (sans fil), et d'autre part au dispositif d'injection 103, par exemple par liaison filaire ou radio. En fonctionnement, l'unité de traitement et de contrôle 105 est adaptée à recevoir les données de glycémie du patient mesurées par le capteur 101, et à commander électriquement le dispositif 103 pour injecter au patient des doses d'insuline déterminées à des instants déterminés. Dans cet exemple, l'unité de traitement et de contrôle 105 est en outre adaptée à recevoir, par l'intermédiaire d'une interface utilisateur non détaillée, des données cho(t) représentatives de l'évolution, en fonction du temps, de la quantité de glucose ingérée par le patient.

L'unité de traitement et de contrôle 105 est adaptée à déterminer les doses d'insuline à injecter au patient en tenant compte notamment de l'historique de glycémie mesurée par le capteur 101, de l'historique d'insuline injectée par le dispositif 103, et de l'historique d'ingestion de glucose par le patient. Pour cela, l'unité de traitement et de contrôle 105 comprend un circuit de calcul numérique (non détaillé), comprenant par exemple un microprocesseur. L'unité de traitement et de contrôle 105 est par exemple un dispositif mobile porté par le patient tout au long de la journée et/ou de la nuit, par exemple un dispositif de type smartphone configuré pour mettre en oeuvre un procédé de régulation du type décrit ci-après.

Dans le mode de réalisation de la figure 1, l'unité de traitement et de contrôle 105 est adaptée à déterminer la quantité d'insuline à administrer au patient en tenant compte d'une prédiction de l'évolution future de sa glycémie en fonction du temps. Plus particulièrement, l'unité de traitement et de contrôle 105 est adaptée, à partir de l'historique d'insuline injectée et de l'historique de glucose ingéré, et en se basant sur un modèle physiologique décrivant l'assimilation de l'insuline par le corps du patient et son impact sur la glycémie, à déterminer une courbe représentative de l'évolution attendue de la glycémie du patient en fonction du temps, sur une période à venir, par exemple une période de 1 à 10 heures. En tenant compte de cette courbe, l'unité de traitement et de contrôle 105 détermine les doses d'insuline à injecter au patient pendant la période à venir, pour que la glycémie réelle (par opposition à la glycémie estimée à partir du modèle physiologique) du patient reste dans des limites acceptables, et en particulier pour limiter les risques d'hyperglycémie ou d'hypoglycémie. Dans ce mode de fonctionnement, comme cela sera expliqué plus en détail ci-après, les données de glycémie réelle mesurées par le capteur 101 sont utilisées principalement à des fins de calibration du modèle physiologique.

La figure 2 est une représentation simplifiée d'un modèle physiologique MPC utilisé dans le système de la figure 1 pour prédire l'évolution future de la glycémie du patient. Sur la figure 2, le modèle est représenté sous la forme d'un bloc de traitement comportant :

une entrée e1 sur laquelle est appliqué un signal i(t) représentatif de l'évolution, en fonction du temps t, de la quantité d'insuline injectée au patient ;

une entrée e2 sur laquelle est appliqué un signal cho(t) représentatif de l'évolution, en fonction du temps t, de la quantité de glucose ingérée par le patient ; et

une sortie s fournissant un signal G(t) représentatif de l'évolution, en fonction du temps t, de la glycémie du patient.

Le modèle physiologique MPC est un modèle compartimental comportant, outre les variables d'entrée i(t) et cho(t) et la variable de sortie G(t), une pluralité de variables d'états correspondant à des variables physiologiques du patient, évoluant en fonction du temps. L'évolution temporelle des variables d'état est régie par un système d'équations différentielles comportant une pluralité de paramètres représentés sur la figure 2 par un vecteur [PARAM] appliqué sur une entrée p1 du bloc MPC. La réponse du modèle physiologique est en outre conditionnée par les états initiaux ou valeurs initiales affectés aux variables d'état, représentés sur la figure 2 par un vecteur [INIT] appliqué sur une entrée p2 du bloc MPC.

La figure 3 est un diagramme représentant plus en détail un exemple (non limitatif) du modèle physiologique MPC utilisé dans le système de la figure 1 pour prédire l'évolution future de la glycémie du patient. Cet exemple de modèle, connu sous le nom de modèle de Hovorka, est décrit plus en détail dans l'article intitulé "Nonlinear model prédictive control of glucose concentration in subjects with type 1 diabètes" de Roman Hovorka et al. (Physiol Meas. 2004;25:905-920), et dans l'article intitulé "Partitioning glucose distribution/transport, disposal, and endogenous production during IVGTT", de Roman Hovorka et al. (Am J Physiol Endocrinol Metab 282: E992-E1007, 2002).

Le modèle physiologique de la figure 3 comprend un premier sous-modèle bi-compartimental 301 décrivant l'effet d'une prise alimentaire de glucose sur le taux d'apparition du glucose dans le plasma sanguin. Le sous-modèle 301 prend pour entrée la quantité de glucose ingérée cho(t), par exemple en mmol/min, et fournit à sa sortie un taux $U_G$ d'absorption du glucose dans le plasma sanguin, par exemple en mmol/min. Le sous-modèle 301 comprend deux variables d'état $D_1$ et $D_2$ correspondant respectivement à des masses de glucose, par exemple en mmol, dans des premier et deuxième compartiments.

Le modèle de la figure 3 comprend en outre un deuxième sous-modèle bi-compartimental 303 décrivant l'absorption, dans le plasma sanguin, de l'insuline administrée au patient. Le sous-modèle 303 prend pour entrée la quantité d'insuline i(t) injectée au patient, par exemple en mU/min, et fournit à sa sortie un taux $U_I$ d'absorption de l'insuline dans le plasma sanguin, par exemple en mU/min. Le sous-modèle 303 comprend deux variables d'état $S_1$ et $S_2$ correspondant respectivement à des masses d'insuline, par exemple en mmol, dans des premier et deuxième compartiments.

Le modèle de la figure 3 comprend de plus un troisième sous-modèle 305 décrivant la régulation du glucose par le corps du patient. Le sous-modèle 305 prend pour entrées les taux d'absorption $U_G$ du glucose et $U_I$ de l'insuline, et fournit à sa sortie la glycémie G(t), c'est-à-dire la concentration en glucose dans le plasma sanguin, par exemple en mmol/l. Le sous-modèle 305 comprend six variables d'état $Q_1$, $Q_2$, $x_3$, $x_1$, $x_2$, I. Les variables $Q1$ et $Q2$ correspondent respectivement à des masses de glucose, par exemple en mmol, dans des premier et deuxième compartiments. Les variables $x_1$, $x_2$, $x_3$ sont des variables sans unité représentant chacune des actions de l'insuline sur la cinétique du glucose. La variable I correspond à l'insulinémie, c'est-à-dire la concentration en insuline dans le plasma sanguin, par exemple en mU/l.

Le modèle de Hovorka est régi par le système d'équations suivant :

$$G(t) = \frac{Q_1(t)}{V_G}$$

$$\frac{dQ_1}{dt} = -\left[\frac{F_{01}^c}{V_G \cdot G(t)} + x_1(t)\right] \cdot Q_1(t) + k_{12}Q_2(t) - F_R + EGP_0 \cdot [1 - x_3(t)] + U_G(t)$$

$$\frac{dQ_2}{dt} = x_1(t) \cdot Q_1(t) - [k_{12} + x_2(t)] \cdot Q_2(t)$$

$$\frac{dx_1}{dt} = -k_{b1} \cdot x_1(t) + k_{a1} \cdot I(t)$$

$$\frac{dx_2}{dt} = -k_{b2} \cdot x_2(t) + k_{a2} \cdot I(t)$$

$$\frac{dx_3}{dt} = -k_{b3} \cdot x_3(t) + k_{a3} \cdot I(t)$$

$$\frac{dS_1}{dt} = i(t) - k_a \cdot S_1(t)$$

$$\frac{dS_2}{dt} = k_a \cdot S_1(t) - k_a \cdot S_2(t)$$

$$\frac{dI}{dt} = \frac{k_a \cdot S_2(t)}{V_I} - k_e \cdot I(t)$$

$$\frac{dD_1}{dt} = cho(t) - \frac{D_1(t)}{t_{max}}$$

$$\frac{dD_2}{dt} = \frac{D_1(t)}{t_{max}} - \frac{D_2(t)}{t_{max}}$$

$$U_G = \frac{D_2(t)}{t_{max}}$$

Avec :

$$F_{01}^c = \frac{F_{01} \cdot G(t)}{0.85 \cdot (G(t) + 1.0)}$$

$$F_R = \begin{cases} R(G - 9) \cdot V_G & si\ G > 9 \\ 0 & sinon \end{cases}$$

Dans ce système d'équations, les grandeurs $V_G$, $F_{01}$, $k_{12}$, $F_R$, $EGP_0$, $k_{b1}$, $k_{a1}$, $k_{b2}$, $k_{a2}$, $k_{b3}$, $k_{a3}$, $k_a$, $V_I$, $k_e$ et $t_{max}$ sont des paramètres. $V_G$ correspond au volume de distribution du glucose, par exemple en litre, $F_{01}$ correspond à un taux de transfert du glucose non insulino-dépendant, par exemple en mmol/min, $k_{12}$ correspond à une constante de taux de transfert entre les deux compartiments du sous modèle 305, par exemple en min$^{-1}$, $k_{a1}$, $k_{a2}$, $k_{a3}$ correspondent à des constantes de taux de désactivation de l'insuline, par exemple en min$^{-1}$, $F_R$ correspond à une excrétion urinaire du glucose, par exemple en mmol/min, $EGP_0$ correspond à une production endogène du glucose, par exemple en min$^{-1}$, $k_{b1}$, $k_{b2}$ et $k_{b3}$ correspondent à des constantes de taux d'activation de l'insuline, par exemple en min$^{-1}$, $k_a$ correspond à une constante de taux d'absorption de l'insuline injectée en sous-cutané, par exemple en min$^{-1}$, $V_I$ correspond au volume de distribution de l'insuline, par exemple en litre, $k_e$ correspond à un taux d'élimination de l'insuline du plasma, par exemple en min$^{-1}$, et $t_{max}$ correspond à un temps passé jusqu'au pic d'absorption du glucose ingéré par le patient, par exemple en min. Ces quinze paramètres correspondent au vecteur [PARAM] de la représentation de la figure 2. Le vecteur [INIT] comprend quant à lui dix valeurs correspondant aux valeurs initiales (à un instant $t_0$ de début d'une phase de simulation du comportement du patient à partir du modèle) affectées aux dix variables d'état $D_1$, $D_2$, $S_1$, $S_2$, $Q_1$, $Q_2$, $x_1$, $x_2$, $x_1$ et I du modèle.

Parmi les paramètres du vecteur [PARAM], certains peuvent être considérés comme constants pour un patient donné. Il s'agit par exemple des paramètres $k_{12}$, $k_{a1}$, $k_{a2}$, $k_{a3}$, $k_a$, $k_e$, $V_I$, $V_G$ et $t_{max}$. D'autres paramètres, appelés ci-après paramètres temps-dépendants, sont en revanche susceptibles d'évoluer dans le temps, par exemples les paramètres $k_{b1}$, $k_{b2}$, $k_{b3}$, $EGP_0$, $F_{01}$ et $F_R$. Du fait de cette variabilité de certains paramètres du système, il est en pratique nécessaire de ré-étalonner ou re-calibrer régulièrement le modèle en cours d'utilisation, par exemple toutes les 1 à 20 minutes, pour s'assurer que les prédictions du modèle restent pertinentes. Cette mise à jour du modèle, aussi appelée personnalisation du modèle, doit pouvoir être réalisée de façon automatique par le système de la figure 1, c'est-à-dire sans qu'il soit nécessaire de mesurer physiquement les paramètres temps-dépendants du système sur le patient puis de les transmettre à l'unité de traitement et de contrôle 105.

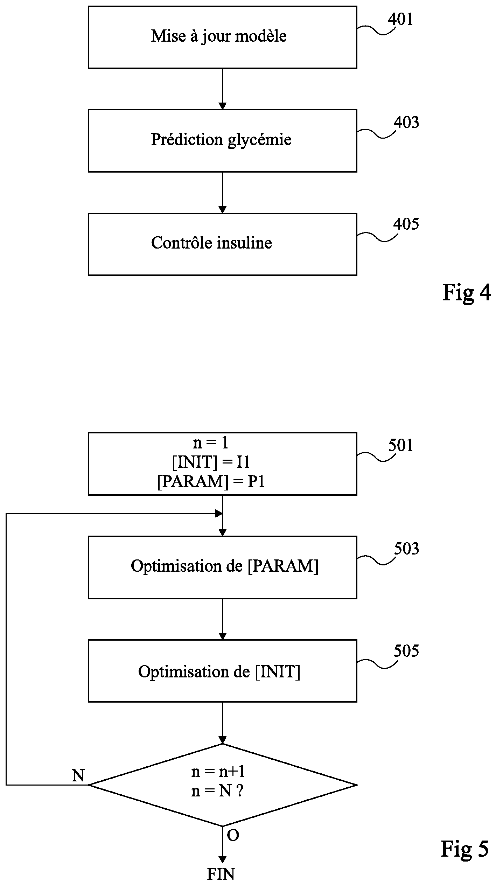

La figure 4 est un diagramme illustrant un exemple d'un procédé automatisé de régulation de glycémie mis en oeuvre par le système de la figure 1. Un tel procédé ne fait pas parti de l'invention revendiquée.

Ce procédé comprend une étape 401 de re-calibration ou mise à jour du modèle, pouvant par exemple être répétées à intervalles réguliers, par exemple toutes les 1 à 20 minutes. Lors de cette étape, l'unité de traitement et de contrôle 105 met en oeuvre un procédé de ré-estimation des paramètres temps-dépendants du modèle en tenant compte des données d'insuline effectivement injectée par le dispositif 103 et des données de glycémie réelle mesurée par le capteur 101 pendant une période d'observation passée, par exemple une période de 1 à 10 heures précédant l'étape de calibration. Plus particulièrement, lors de l'étape de calibration, l'unité de traitement et de contrôle 105 simule le comportement du patient sur la période d'observation passée à partir du modèle physiologique (en tenant compte des éventuelles ingestions de glucose et injections d'insuline pendant cette période), et compare la courbe de glycémie estimée par le modèle à la courbe de glycémie réelle mesurée par le capteur pendant cette même période. L'unité de traitement et de contrôle 105 recherche alors, pour les paramètres temps-dépendants du modèle, un jeu de valeurs conduisant à minimiser une grandeur représentative de l'erreur entre la courbe de glycémie estimée par le modèle et la courbe de glycémie réelle pendant la période d'observation. A titre d'exemple, l'unité de traitement et de contrôle recherche un jeu de paramètres conduisant à minimiser un indicateur m représentatif de l'aire entre la courbe de glycémie estimée par le modèle et la courbe de glycémie réelle pendant la période d'observation, par exemple défini comme suit :

$$m = \frac{1}{\Delta T} \sum_{t=t_0}^{t_0+\Delta T} |g(t) - \hat{g}(t)|^2$$

où t est la variable temps discrétisée, $t_0$ correspond à l'instant de début de la phase d'observation passé, $t_0+\Delta T$ correspond à l'instant de fin de la phase d'observation passée (correspondant par exemple à l'instant de début de l'étape de calibration du modèle), g est la courbe d'évolution temporelle de la glycémie réelle mesurée par le capteur 101 pendant la période $[t0, t_0+\Delta T]$, et $\hat{g}$ est la courbe de glycémie estimée à partir du modèle pendant la période $[t_0, t_0+\Delta T]$. L'algorithme de recherche de paramètres optimaux utilisé lors de cette étape n'est pas détaillé dans la présente demande, les modes de réalisation décrits étant compatibles avec les algorithmes usuels utilisés dans des domaines variés pour résoudre des problèmes d'optimisation de paramètres par minimisation d'une fonction de coût.

Le procédé de la figure 4 comprend en outre, après l'étape 401, une étape 403 de prédiction, par l'unité de traitement et de contrôle 105, de l'évolution temporelle de la glycémie du patient sur une période à venir, à partir du modèle physiologique mis à jour à l'étape 401 et en tenant compte de l'historique d'insuline injectée au patient et de l'historique de glucose ingéré par le patient.

Le procédé de la figure 4 comprend de plus, après l'étape 403, une étape 405 de détermination, par l'unité de traitement et de contrôle 105, en tenant compte de la courbe de glycémie future prédite à l'étape 403, des doses d'insuline à injecter au patient pendant une période à venir. A l'issue de cette étape, l'unité de traitement et de contrôle 105 peut programmer le dispositif d'injection 103 pour administrer les doses déterminées pendant la période à venir.

Les étapes 403 de prédiction de la glycémie et 405 et de détermination des doses futures d'insuline à administrer peuvent par exemple être réitérées à chaque mise à jour du modèle physiologique (c'est-à-dire après chaque itération de l'étape 401), à chaque nouvelle ingestion de glucose signalée par le patient, et/ou à chaque nouvelle administration d'une dose d'insuline par le dispositif d'injection 103.

Un problème qui se pose dans le fonctionnement décrit ci-dessus est que, lors de la mise à jour du modèle physiologique à l'étape 401, l'unité de traitement et de contrôle 105 doit définir un vecteur [INIT] d'états initiaux (états à $t_0$) des variables d'état du modèle, pour pouvoir simuler le comportement du patient à partir du modèle. Ces états initiaux sont nécessaires non seulement pour pouvoir prédire l'évolution future de la glycémie du patient (étape 403), mais aussi pendant l'étape de mise à jour du modèle elle-même (étape 401), pour pouvoir simuler l'évolution de la glycémie du patient pendant la période d'observation passée, de façon à pouvoir comparer la glycémie simulée à la glycémie mesurée.

Pour définir les états initiaux des variables d'état du modèle, une première possibilité consiste à faire l'hypothèse que, dans la période précédant la période d'observation $[t0, t_0+\Delta T]$ sur laquelle est basée la calibration du modèle, le patient se trouvait dans un état stationnaire, avec un débit d'insuline injectée constant, et une prise alimentaire de glucose nulle. Sous cette hypothèse, toutes les dérivées du système d'équations différentielles peuvent être considérées comme nulles à l'instant initial $t_0$. Les valeurs à $t_0$ des variables d'état du système peuvent alors être calculées analytiquement. Un inconvénient de cette solution est que la sortie du modèle (la glycémie estimée) n'est pas contrainte. En particulier, la glycémie estimée à l'instant $t_0$ peut être différente de la glycémie réelle mesurée à l'instant $t_0$. Dans ce

cas, l'algorithme mis en oeuvre à l'étape 401 de recherche des paramètres temps-dépendants du modèle par minimisation de l'erreur entre la glycémie simulée et la glycémie mesurée peut avoir du mal à converger.

Pour améliorer l'initialisation, une deuxième possibilité consiste à faire les mêmes hypothèses que précédemment, mais en contraignant la variable $Q_1(t_0)$ de sorte que la glycémie estimée à l'instant $t_0$ soit égale à la glycémie réelle mesurée par le capteur. Ceci permet d'améliorer la pertinence de l'initialisation à l'instant $t_0$. Toutefois, à l'instant $t_0$, la dérivée de la glycémie estimée et la dérivée de la glycémie réelle peuvent diverger. En conséquence, l'algorithme de recherche des paramètres temps-dépendants du système peut là encore avoir encore du mal à converger.

En pratique, les deux méthodes susmentionnées de détermination des états initiaux du modèle physiologique sont souvent insatisfaisantes, ce qui rend difficile la recherche d'un jeu de valeurs pertinent pour les paramètres temps-dépendants du modèle. Une conséquence est que les prévisions de l'évolution future de la glycémie du patient à partir du modèle peuvent être erronées, et conduire à une mauvaise régulation de la glycémie par le système.

Pour pallier ce problème, selon l'invention, on prévoit, lors de la phase de calibration ou de mise à jour du modèle (étape 401), de considérer les états initiaux [INIT] du modèle comme des variables aléatoires, et d'effectuer, comme cela est fait pour estimer les paramètres temps-dépendants du modèle, une recherche d'un jeu optimal de valeurs d'états initiaux par minimisation d'une grandeur représentative de l'erreur entre la courbe de glycémie estimée à partir du modèle et la courbe de glycémie réelle pendant la période d'observation sur laquelle est basée la calibration.

Si le nombre cumulé des paramètres temps-dépendants et des variables d'états du modèle physiologique est suffisamment faible, les valeurs optimales des paramètres temps-dépendants et des états initiaux des variables d'état peuvent être déterminées simultanément, lors d'une même étape d'optimisation du modèle par minimisation de l'erreur entre la glycémie estimée et la glycémie réelle sur la période d'observation passée.

En pratique, dans le modèle de Hovorka, ainsi que dans la plupart des modèles physiologiques décrivant l'assimilation de l'insuline et du glucose par le corps et leur impact sur la glycémie, le nombre cumulé des paramètres temps-dépendants et des variables d'états est relativement important, ce qui peut conduire à une instabilité numérique lors de la phase de recherche des valeurs optimales. Autrement dit, certaines valeurs peuvent être difficiles voire impossibles à estimer en une seule recherche, le nombre d'inconnues étant trop important. Dans ce cas, et dans le contexte de l'invention, le problème peut donc être décomposé en deux sous-problèmes, correspondant respectivement à l'estimation des paramètres temps-dépendants du modèle et à l'estimation des états initiaux du modèle, comme cela va maintenant être décrit en relation avec la figure 5.

La figure 5 est un diagramme illustrant un exemple d'un mode de réalisation d'un procédé automatisé de calibration ou de mise à jour du système de la figure 1, correspondant à un exemple de mise en oeuvre de l'étape 401 de la figure 4.

Ce procédé comprend une étape 501 au cours de laquelle le vecteur de paramètres [PARAM] (réduit ici aux seuls paramètres temps-dépendants du modèle) est initialisé à un premier jeu de valeurs P1. Le jeu P1 correspond par exemple aux valeurs prises par les paramètres [PARAM] avant le début de la phase de mise à jour du modèle. A titre de variante, le jeu de valeurs P1 est un jeu de référence prédéterminé correspondant par exemple aux valeurs moyennes prises par les paramètres [PARAM] sur une période de référence. Lors de l'étape 501, le vecteur d'états initiaux [INIT] des variables d'état est en outre initialisé à un premier jeu de valeur I1. Le jeu de valeurs I1 est par exemple déterminé analytiquement comme décrit ci-dessus, en faisant l'hypothèse d'un état stationnaire du patient dans la période précédant la phase de calibration, et en faisant coïncider la glycémie estimée à l'instant $t_0$ et la glycémie réelle mesurée à ce même instant.

Lors d'une étape 503 postérieure à l'étape 501, l'unité de traitement et de contrôle 105 recherche, en fixant le jeu d'états initiaux [INIT] à son état courant, un jeu de valeurs des paramètres temps-dépendants du modèle conduisant à minimiser une grandeur représentative de l'erreur entre la courbe de glycémie estimée à partir du modèle et la courbe de glycémie réelle pendant la période d'observation, par exemple l'indicateur m défini ci-dessus. A l'issue de cette étape, le vecteur [PARAM] est mis à jour avec les nouvelles valeurs estimées.

Lors d'une étape 505 postérieure à l'étape 503, l'unité de traitement et de contrôle 105 recherche, en fixant le jeu de paramètres [PARAM] à son état courant, un jeu de valeurs d'états initiaux des variables d'état conduisant à minimiser une grandeur représentative de l'erreur entre la courbe de glycémie estimée à partir du modèle et la courbe de glycémie réelle pendant la période d'observation, par exemple l'indicateur m défini ci-dessus, ou tout autre indicateur représentatif de l'erreur entre les deux courbes, par exemple un indicateur basé sur la norme L1. A l'issue de cette étape, le vecteur [INIT] est mis à jour avec les nouvelles valeurs estimées.

Dans le contexte de l'invention, les étapes 503 et 505 sont réitérées un nombre N prédéterminé de fois, où N est un entier supérieur à 1. Les valeurs des paramètres temps-dépendants et des états initiaux du modèle mis à jour correspondent alors aux valeurs des vecteurs [PARAM] et [INIT] à l'issue de la Nième itération des étapes 503 et 505. A titre de variante le nombre d'itérations des étapes 503 et 505 peut ne pas être prédéterminé, et être ajusté en tenant compte de l'évolution de l'indicateur m d'erreur entre la glycémie estimée à partir du modèle et la glycémie réelle sur la période d'observation.

Les algorithmes de recherche de valeurs optimales utilisés lors des étapes 503 et 505 ne sont pas détaillés dans la présente demande, les modes de réalisation décrits étant compatibles avec les algorithmes usuels utilisés dans des domaines variés pour résoudre des problèmes d'optimisation de paramètres par minimisation d'une fonction de coût.

Un avantage du mode de fonctionnement décrit ci-dessus, dans lequel les valeurs initiales des variables d'état du modèle physiologique sont déterminées par minimisation d'une grandeur représentative de l'erreur entre les données de glycémie mesurée et la glycémie estimée pendant une période d'observation passée, est qu'il permet d'améliorer la qualité de la prédiction de la glycémie future du patient, et ainsi de contrôler avec une plus grande pertinence les apports en insuline.

Un objet d'un autre mode de réalisation non couvert par l'invention revendiquée est de permettre de limiter les risques pour le patient liés à une éventuelle défaillance du modèle physiologique utilisé pour prédire la glycémie future du patient.

Pour cela, selon un aspect d'un mode de réalisation, le dispositif de contrôle et de traitement 105 du système de régulation est adapté, après chaque mise à jour ou re-calibration du modèle physiologique (étape 401), à estimer la qualité du modèle physiologique mis à jour au moyen d'un ou plusieurs indicateurs numériques de qualité, et, si la qualité du modèle est jugée insatisfaisante, à cesser d'utiliser le modèle pour réguler la glycémie du patient.

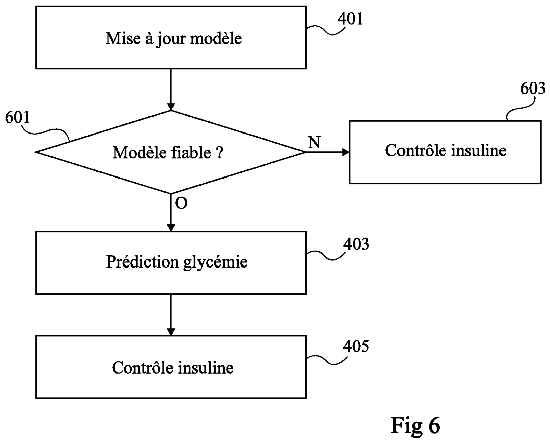

La figure 6 est un diagramme illustrant un exemple d'un mode de réalisation d'un procédé automatisé de régulation de glycémie mis en oeuvre par le système de la figure 1. Un tel procédé ne fait pas parti de l'invention revendiquée.

Ce procédé comprend les mêmes étapes 401, 403 et 405 que dans l'exemple de la figure 4. Toutefois, le procédé de la figure 6 comprend en outre, après chaque étape 401 de mise à jour du modèle physiologique exploité par le système de régulation et avant la mise en oeuvre des étapes suivantes 403 de prédiction de la glycémie future du patient à partir du modèle et 405 de contrôle de la délivrance d'insuline à partir de la prédiction de glycémie, une étape 601 de vérification de la qualité du modèle mis à jour.

Lors de l'étape 601, l'unité de traitement et de contrôle 105 détermine un ou plusieurs indicateurs numériques de la qualité du modèle mis à jour à l'étape 401. A titre d'exemple, l'unité de traitement et de contrôle calcule un indicateur numérique de qualité représentatif de l'aire entre la courbe de glycémie estimée à partir du modèle et la courbe de glycémie réelle mesurée par le capteur 101 pendant une période d'observation passée. Cet indicateur correspond par exemple à la grandeur m définie ci-dessus.

A la place, ou en complément, d'un indicateur représentatif de la surface entre les courbes de glycémie estimée et de glycémie réelle pendant une période d'observation passée, l'unité de traitement et de contrôle 105 peut calculer l'un et/ou l'autre des indicateurs de qualité $m_1$ et $m_2$ suivants :

$$m_1(t_{courant}) = g(t_{courant}) - \hat{g}(t_{courant})$$

$$m_2(t_{courant}) = g'(t_{courant}) - \hat{g}'(t_{courant}),$$

où $t_{courant}$ désigne un instant présent de mise en oeuvre de l'étape 601 de vérification de la qualité du modèle, g correspond à la fonction d'évolution temporelle de la glycémie réelle mesurée par le capteur 101, $\hat{g}$ correspond à la fonction d'évolution temporelle de la glycémie simulée à partir du modèle, g' correspond à la dérivée de la fonction d'évolution temporelle de la glycémie réelle, et $\hat{g}'$ correspond à la dérivée de la fonction d'évolution temporelle de la glycémie simulée.

A titre d'exemple, la qualité du modèle peut être considérée comme satisfaisante par l'unité de traitement et de contrôle 105 lorsque les valeurs m, $m_1$ et $m_2$ sont inférieures à des seuils prédéfinis. Plus généralement, tout autre critère de qualité ou toute autre combinaison de critères de qualité peuvent être utilisés à l'étape 601 pour déterminer si le modèle physiologique re-calibré à l'étape 401 peut être considéré comme fiable.

Si le modèle physiologique est considéré comme fiable à l'étape 601 (O), les étapes 403 et 405 peuvent être mises en oeuvre de façon similaire à ce qui a été décrit précédemment, c'est-à-dire que l'unité de traitement et de contrôle 105 continue de se baser sur les prédictions réalisées par le modèle physiologique pour réguler l'administration de l'insuline au patient.

Si le modèle physiologique est jugé insuffisamment fiable à l'étape 601 (N), l'unité de traitement et de contrôle 105 cesse d'utiliser ce modèle pour réguler l'administration de l'insuline au patient, et met en oeuvre une méthode de régulation de substitution lors d'une étape 603.

A titre d'exemple, lors de l'étape 603, l'unité de traitement et de contrôle 105 utilise un modèle physiologique simplifié, par exemple un modèle compartimental comportant un nombre de variables d'état et un nombre de paramètres réduit par rapport au modèle initial, pour prédire l'évolution de la glycémie du patient et réguler en conséquence l'injection d'insuline.

A titre de variante, lors de l'étape 603, l'unité de traitement et de contrôle 105 cesse de mettre en oeuvre une commande prédictive, c'est-à-dire qu'elle cesse d'utiliser un modèle physiologique pour prédire la glycémie future du patient et réguler en conséquence l'injection d'insuline. Dans ce cas, l'unité de traitement et de contrôle 105 commande par exemple le dispositif d'injection d'insuline 103 pour administrer des doses préprogrammées d'insuline, correspondant par exemple à un débit basal de référence prescrit au patient.

Une telle méthode de substitution peut par exemple être utilisée pendant une période de temps prédéterminée. A l'issue de cette période, les étapes 401 de calibration du modèle physiologique principal et 601 d'estimation de la qualité du modèle physiologique principal peuvent être réitérées, pour, si la qualité du modèle physiologique principal est jugée satisfaisante, réactiver l'utilisation de ce modèle pour réguler l'administration de l'insuline au patient.

On notera que le procédé de la figure 6 ne se limite pas au mode de réalisation décrit en relation avec les figures 4 et 5, dans lequel la calibration du modèle physiologique comprend une étape de détermination des valeurs initiales des variables d'état du modèle par minimisation d'une grandeur représentative de l'erreur entre les données de glycémie mesurée et la glycémie estimée pendant une période d'observation, mais peut être utilisé quelle que soit la méthode choisie de détermination des valeurs initiales des variables d'état du modèle.

L'invention est définie par les revendications jointes.

Des modes de réalisation particuliers ont été décrits. Diverses variantes et modifications apparaîtront à l'homme de l'art. En particulier, les modes de réalisation décrits ne se limitent pas à l'exemple particulier de modèle physiologique détaillé dans la présente description, à savoir le modèle de Hovorka, mais sont compatible avec tout modèle physiologique décrivant l'assimilation de l'insuline par le corps d'un patient et son effet sur la glycémie du patient, par exemple le modèle dit de Cobelli, décrit dans l'article intitulé "A System Model of Oral Glucose Absorption: Validation on Gold Standard Data", de Chiara Dalla Man et al. (IEEE TRANSACTIONS ON BIOMEDICAL ENGINEERING, VOL. 53, NO. 12, DECEMBER 2006) .

## Revendications

1. Système automatisé de régulation de la glycémie d'un patient, comportant :

   un capteur de glycémie (101) ;
   un dispositif d'injection d'insuline (103) ; et
   une unité de traitement et de contrôle (105) adaptée à prédire l'évolution future de la glycémie du patient à partir d'un modèle physiologique et à commander le dispositif d'injection d'insuline (103) en tenant compte de cette prédiction,
   dans lequel :

   le modèle physiologique comporte un système d'équations différentielles décrivant l'évolution d'une pluralité de variables d'état ($D_1$, $D_2$, $S_1$, $S_2$, $Q_1$, $Q_2$, $x_1$, $x_2$, $x_3$, I) en fonction du temps ; et
   l'unité de traitement et de contrôle (105) est adaptée à mettre en oeuvre une étape de calibration automatique du modèle physiologique comprenant une étape d'estimation de valeurs initiales ([INIT]) des variables d'état par minimisation d'une grandeur représentative de l'erreur, pendant une période d'observation passée, entre la glycémie estimée à partir du modèle physiologique et la glycémie mesurée par le capteur (101), dans lequel l'étape de calibration automatique du modèle physiologique comprend en outre une étape d'estimation de paramètres ([PARAM]) du système d'équations différentielles par minimisation de ladite grandeur,
   et dans lequel l'étape de calibration automatique du modèle physiologique comprend une pluralité d'itérations successives des étapes a) et b) suivantes :

   a) estimer les paramètres ([PARAM]) du système d'équations différentielles par minimisation de ladite grandeur en fixant les valeurs initiales ([INIT]) des variables d'état ; et
   b) estimer les valeurs initiales ([INIT]) des variables d'état par minimisation de ladite grandeur en fixant les paramètres ([PARAM]) du système d'équations différentielles.

2. Système selon la revendication 1, dans lequel ladite grandeur est représentative de l'aire entre une première courbe g représentative de l'évolution temporelle de la glycémie estimée à partir du modèle sur la période d'observation, et une deuxième courbe g représentative de l'évolution temporelle de la glycémie mesurée par le capteur (101) sur la période d'observation.

3. Système selon la revendication 2, dans lequel ladite grandeur est définie comme suit :

$$m = \frac{1}{\Delta T} \sum_{t=t_0}^{t_0 + \Delta T} |g(t) - \hat{g}(t)|^2$$

où t est une variable temps discrétisée, $t_0$ est l'instant de début de la phase d'observation, et $t_0+\Delta T$ est l'instant de fin de la phase d'observation.

4. Système selon l'une quelconque des revendications 1 à 3, dans lequel, à la première itération de l'étape a), les valeurs initiales (INIT]) des variables d'état sont déterminées analytiquement en faisant l'hypothèse que toutes les dérivées du système d'équations différentielles sont nulles.

5. Système selon l'une quelconque des revendications 1 à 4, dans lequel, pour simuler l'évolution de la glycémie du patient à partir du modèle physiologique, l'unité de traitement et de contrôle (105) tient compte de l'historique d'insuline (i(t)) injectée au patient par le dispositif d'injection (103) et de l'historique de glucose (cho(t)) ingéré par le patient.

6. Système selon l'une quelconque des revendications 1 à 5, dans lequel le modèle physiologique est le modèle de Hovorka.

**Patentansprüche**

1. Automatisiertes System zur Überwachung des Blutzuckerspiegels eines Patienten, das Folgendes aufweist:

einen Blutzuckersensor (101);
eine Insulininjektionsvorrichtung (103); und
eine Überwachungs- und Steuereinheit (105), die eingerichtet ist, die zukünftige Entwicklung des Blutzuckerspiegels des Patienten anhand eines physiologischen Modells vorherzusagen und die Insulininjektionsvorrichtung (103) auf der Grundlage dieser Vorhersage zu überwachen,
wobei:

das physiologische Modell ein System von Differentialgleichungen aufweist, das die zeitliche Entwicklung einer Vielzahl von Zustandsvariablen ($D_1$, $D_2$, $S_1$, $S_2$, $Q_1$, $Q_2$, $X_1$, $X_2$, $X_3$, I) beschreibt; und
die Überwachungs- und Steuereinheit (105) eingerichtet ist, einen Schritt automatischer Kalibrierung des physiologischen Modells zu implementieren, der einen Bewertungsschritt von Anfangswerten ([INIT]) der Zustandsvariablen durch Minimierung eines Wertes aufweist, der die Diskrepanz zwischen dem geschätzten Blutzuckerspiegel, wie vom physiologischen Modell geschätzt, und dem Blutzuckerspiegel, wie vom Sensor (101) für einen vergangenen Beobachtungszeitraum gemessen, darstellt,
wobei der Schritt zur automatischen Kalibrierung des physiologischen Modells ferner einen Bewertungsschritt von Parametern ([PARAM]) des Systems von Differentialgleichungen durch Minimierung des Wertes aufweist, und wobei
der Schritt der automatischen Kalibrierung des physiologischen Modells eine Vielzahl von aufeinanderfolgenden Iterationen der folgenden Schritte a) und b) aufweist:

a) Bewertung der Parameter ([PARAM]) des Differentialgleichungssystems durch Minimierung des genannten Wertes durch Festlegen der Anfangswerte ([INIT]) der Zustandsvariablen; und
b) Bewertung der Anfangswerte ([INIT]) der Zustandsvariablen durch Minimierung dieser Werte durch Festlegung der Parameter ([PARAM]) des Differentialgleichungssystems.

2. System nach Anspruch 1, wobei der Wert repräsentativ für die Fläche zwischen einer ersten Kurve $\hat{g}$, die die zeitliche Entwicklung des Blutzuckerspiegels repräsentiert, und zwar wie vom Modell für die Beobachtungsperiode geschätzt, und einer zweiten Kurve g ist, die die zeitliche Entwicklung des Blutzuckerspiegels repräsentiert, und zwar wie vom Sensor (101) für die Beobachtungsperiode gemessen.

3. System nach Anspruch 2, wobei der Wert definiert ist als:

$$m = \frac{1}{\Delta T} \sum_{t=t_0}^{t_0 + \Delta T} |g(t) - \hat{g}(t)|^2$$

wobei t eine diskretisierte Zeitvariable ist, wobei $t_0$ die Anfangszeit des Beobachtungsschritts ist, und wobei $t_0 + \Delta T$ die Endzeit des Beobachtungsschritts ist.

4. System nach einem der Ansprüche 1 bis 3, wobei bei der ersten Iteration von Schritt a) die Anfangswerte ([INIT]) der Zustandsvariablen analytisch berechnet werden unter der Hypothese, dass alle Ableitungen des Differentialgleichungssystems Null sind.

5. System nach einem der Ansprüche 1 bis 4, wobei die Überwachungs- und Steuereinheit (105) zur Simulation der Entwicklung des Blutzuckerspiegels des Patienten auf der Grundlage des physiologischen Modells den Verlauf des dem Patienten durch die Injektionsvorrichtung (103) injizierten Insulins (i(t)) und den Verlauf der vom Patienten aufgenommenen Glukose (cho(t)) berücksichtigt.

6. System nach einem der Ansprüche 1 bis 5, wobei das physiologische Modell das Hovorka-Modell ist.

**Claims**

1. Automated system for monitoring the blood sugar level of a patient, comprising:

   a blood sugar sensor (101);
   an insulin injection device (103); and
   a monitoring and control unit (105) configured to foresee the future evolution of the blood sugar level of the patient from a physiological model and to monitor the insulin injection device (103) based on this prediction, wherein:

   the physiological model comprises a system of differential equations that describes the evolution in time of a plurality of state variables ($D_l$, $D_2$, $S_1$, $S_2$, $Q_1$, $Q_2$, $X_1$, $X_2$, $X_3$, I); and
   the monitoring and control unit (105) is configured to implement a step of automatic calibration of the physiological model comprising a step of assessment of initial values ([INIT]) of the state variables by minimization of a value representative of the discrepancy between the estimated blood sugar level as estimated from the physiological model and the blood sugar level as measured by the sensor (101) for a past observation period,
   wherein the step of automatic calibration of the physiological model further comprises a step of assessment of parameters ([PARAM]) of the system of differential equations by minimization of said value, and wherein the step of automatic calibration of the physiological model comprises a plurality of successive iterations of the following steps a) and b) :

   a) assessment of the parameters ([PARAM]) of the system of differential equations by minimization of said value by fixing the initial values ([INIT]) of the state variables; et
   b) assessment of the initial values ([INIT]) of the state variables by minimization of said values by fixing the parameters ([PARAM]) of the system of differential equations.

2. System according to claim 1, wherein said value is representative of the surface between a first curve $\hat{g}$ that represents the evolution in time of the blood sugar level as estimated from the model for the observation period, and a second curve g that represents the evolution in time of the blood sugar level as measured by the sensor (101) for the observation period.

3. System according to claim 2, wherein said value is defined as:

$$m = \frac{1}{\Delta T} \sum_{t=t_0}^{t_0 + \Delta T} |g(t) - \hat{g}(t)|^2$$

where t is a discretized time variable, $t_0$ is the start time of the observation step, and $t_0 + \Delta T$ is the end time of the observation step.

4. System according to any one of claims 1 to 3 wherein, at the first iteration of step a), the initial values ([INIT]) of the state variables are analytically calculated under the hypothesis that all derivatives of the system of differential equations are zero.

5. System according to any one of claims 1 to 4 wherein, to simulate the evolution of the blood sugar level of the patient based on the physiological model, the monitoring and control unit (105) takes the history of insulin (i(t)) injected to the patient by the injection device (103) and the history of glucose (cho(t)) ingested by the patient into account.

6. System according to any one of claims 1 to 5, wherein the physiological model is the Hovorka model.

**Fig 1**

**Fig 2**

**Fig 3**

```
┌─────────────────────────────┐
│      Mise à jour modèle      │ ⌇ 401
└─────────────────────────────┘
              │
              ▼
┌─────────────────────────────┐
│      Prédiction glycémie      │ ⌇ 403
└─────────────────────────────┘
              │
              ▼
┌─────────────────────────────┐
│       Contrôle insuline       │ ⌇ 405
└─────────────────────────────┘
```

**Fig 4**

```
┌─────────────────────────────┐
│           n = 1             │
│        [INIT] = I1          │ ⌇ 501
│       [PARAM] = P1          │
└─────────────────────────────┘
              │
              ▼
┌─────────────────────────────┐
│    Optimisation de [PARAM]   │ ⌇ 503
└─────────────────────────────┘
              │
              ▼
┌─────────────────────────────┐
│     Optimisation de [INIT]    │ ⌇ 505
└─────────────────────────────┘
              │
              ▼
         n = n+1
    N ◇  n = N ?
              │ O
              ▼
            FIN
```

**Fig 5**

```
┌─────────────────────────┐
│                         │      401
│    Mise à jour modèle   │  ⌇
│                         │
└─────────────────────────┘
            │
            ▼
                                                        603
   601              ◇                          ┌─────────────────────────┐
    ⌇        ╱          ╲         N            │                         │
   ────── ◇   Modèle fiable ?  ◇ ──────────▶   │    Contrôle insuline    │
           ╲          ╱                        │                         │
               ◇                              └─────────────────────────┘
            │
            │ O
            ▼
┌─────────────────────────┐
│                         │      403
│   Prédiction glycémie   │  ⌇
│                         │
└─────────────────────────┘
            │
            ▼
┌─────────────────────────┐
│                         │      405
│    Contrôle insuline    │  ⌇
│                         │
└─────────────────────────┘
```

**Fig 6**

17

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- GB 2436873 A

**Littérature non-brevet citée dans la description**

- **ROMAN HOVORKA et al.** Nonlinear model prédictive control of glucose concentration in subjects with type 1 diabètes. *PHYSIOLOGICAL MEASUREMENT,* Août 2004, vol. 25 (4), ISSN 0967-3334, 905-920
- **ROMAN HOVORKA et al.** Nonlinear model prédictive control of glucose concentration in subjects with type 1 diabètes. *Physiol Meas.,* 2004, vol. 25, 905-920

- **ROMAN HOVORKA et al.** Partitioning glucose distribution/transport, disposal, and endogenous production during IVGTT. *Am J Physiol Endocrinol Metab,* 2002, vol. 282, E992-E1007
- **CHIARA DALLA MAN et al.** A System Model of Oral Glucose Absorption: Validation on Gold Standard Data. *IEEE TRANSACTIONS ON BIOMEDICAL ENGINEERING,* Décembre 2006, vol. 53 (12